# EUROPEAN PATENT APPLICATION

(11) **EP 2 614 793 A1**
(43) Date of publication of application: **17.07.2013**
(21) Application number: 12192279.3
(22) Date of filing: 02.03.2005
(51) Int. Cl.: A61F 2/06, A61F 2/07, A61F 2/82

(54) **Medical devices including metallic films and methods for making same**

(30) Priority: 02.03.2004 US 549287 P; 29.12.2004 US 25867; 29.12.2004 US 25866; 29.12.2004 US 25464; 29.12.2004 US 25158; 29.12.2004 US 25660; 29.12.2004 US 25860; 29.12.2004 US 25684
(62) Divisional of application: 05724666.2
(71) Applicant: Boston Scientific Scimed, Inc., Maple Grove, MN 55311-1566 (US)
(72) Inventor: Molaei, Masoud, Mountain View, CA California 94040 (US); Peckham, John, Sunnyvale, CA California 94085 (US); Leynov, Alexander, Walnut Creek, CA California 94597 (US); Porter, Stephen, Christopher, Oakland, CA California 94618 (US); Obara, Robert, Z., Fremont, CA California 94538 (US); Abrams, Robert, M., Los Gatos, CA California 95032 (US)
(74) Representative: Schley, Jan Malte

(57) **Abstract**

Medical devices, such as endoprostheses (100), and methods of making the devices are disclosed. The medical device can include a composite cover (54) formed of a deposited metallic film and one or more polymer layers (101). The polymer layers contribute to mechanical or biological properties of the endoprosthesis.

## Description

### RELATED APPLICATIONS

This application claims the benefit of U.S. provisional patent application no. 60/549,287, filed March 2, 2004, which application is incorporated by reference herein.

### FIELD OF THE INVENTION

The invention relates to medical devices, such as endoprostheses, and methods of making the devices.

### BACKGROUND

The body includes various passageways such as arteries, other blood vessels, and other body lumens. These passageways sometimes become occluded or weakened. For example, the passageways can be occluded by a tumor, restricted by plaque, or weakened by an aneurysm. When this occurs, the passageway can be reopened or reinforced, or even replaced, with a medical endoprosthesis. An endoprosthesis is typically a tubular member that is placed in a lumen in the body. Endoprostheses can be delivered inside the body by a catheter that supports the endoprosthesis in a compacted or reduced-size form as the endoprosthesis is transported to a desired site. Upon reaching the site, the endoprosthesis is expanded, for example, so that it can contact the walls of the lumen.

The expansion mechanism may include forcing the endoprosthesis to expand radially. For example, the expansion mechanism can include the catheter carrying a balloon, which carries a balloon-expandable endoprosthesis. The balloon can be inflated to deform and to fix the expanded endoprosthesis at a predetermined position in contact with the lumen wall. The balloon can then be deflated, and the catheter withdrawn.

In another delivery technique, the endoprosthesis is formed of an elastic material that can be reversibly compacted and expanded, e.g., elastically or through a material phase transition. During introduction into the body, the endoprosthesis is restrained in a radially compacted condition. Upon reaching the desired implantation site, the restraint is removed, for example, by retracting a restraining device such as an outer sheath, enabling the endoprosthesis to self-expand by its own internal elastic restoring force.

### SUMMARY OF THE INVENTION

The invention relates to medical devices, such as endoprostheses, and methods of making the devices. Exemplary endoprostheses include stents, covered stents, and stent-grafts.

In some embodiments, an endoprosthesis includes a tubular framework having first and second ends and a deposited metallic film generally coextensive with at least a portion of the framework. The deposited metallic film may include nickel and titanium. The film may have a thickness of less than about 50 µm. A polymer, e.g., a polymer layer, secures the tubular framework and the deposited metallic film together.

The endoprosthesis may include a plurality of polymer layers. Each polymer layer may have a configuration generally aligned with a portion of the tubular framework. The framework may include a plurality of framework members. The polymer layers may envelope at least some of the framework members and at least a portion of the metallic film.

In some embodiments, an endoprosthesis includes a tubular framework having first end and second ends and a deposited metallic film generally coextensive with at least a portion of the framework. The deposited metallic film may include nickel and titanium. The film may have a thickness of less than about 50 µm. At least one polymer strand extends circumferentially around the endoprosthesis.

A plurality of polymer strands may each extend circumferentially around the endoprosthesis. The polymer strands may define a helical lattice.

The endoprosthesis may exert a radial expansive force when deployed within a body passage with the at least one polymer strand contributing at least a portion of the radial expansive force.

The polymer of the strand may include a derivative of butyric acid and/or a copolymer of urethane and silicone.

In some embodiments, an endoprosthesis includes a tubular member. At least a central portion of the tubular member includes a plurality of plates connected by struts. Each of the plates may be movable with respect to at least another plate. When the endoprosthesis is radially compressed for delivery along a body passage, at least some of the plates may overlap another plate. When the endoprosthesis is radially expanded within a body passage, an extent of the overlap may decreases.

The plates may include a deposited metallic film, e.g., a film including nickel and titanium.

In some embodiments, an endoprosthesis is configured to be deployed within a body passage by using a deployment device. The endoprosthesis is radially compressed within the deployment device and relatively radially expanded within the body passage. The endoprosthesis includes a deposited metallic film having a plurality of fenestrations. The fenestrations have a lower stress in the radially compressed state than in the relatively radially expanded state.

The endoprosthesis may define a longitudinal axis. Each fenestration, in the radially compressed state, may have a generally slit-like shape defined by a plurality of walls extending generally parallel to the longitudinal axis. In the radially expanded state, at least some of the walls of each fenestration may define an angle with respect to the longitudinal axis. At least some of the walls may remain generally parallel with the longitudinal axis.

In some embodiments, an endoprosthesis includes a framework, e.g., a stent body, and a cover comprising a deposited metallic film. A polymer layer is in contact with, e.g., adhered to, at least a portion of the metallic film. The polymer layer can reduce a tendency of the metallic film to tear during handling, e.g., during loading and/or deployment. The polymer layer can enhance an abrasion resistance of the film during handling. The polymer layer may be lubricious.

In one aspect, the invention features an endoprosthesis including a metallic film, e.g., a vapor deposited film, including nickel, titanium, and chromium. A ratio of a weight of chromium of the metallic film to a combined weight of nickel, titanium, and chromium of the metallic film is at least 0.001 and can be less than 0.0075.

Other aspects, features, and advantages of the invention will be apparent from the description of the preferred embodiments thereof and from the claims.

### BRIEF DESCRIPTION OF THE FIGURES

Fig. 1a is a side view of an endoprosthesis in the radially expanded state as deployed within a body passage adjacent an aneurysm. The endoprosthesis has a plurality of polymer layers.
Fig. 1b is a cross-section through the endoprosthesis of Fig. 1a.
Fig. 2a is a side view of a distal portion of a deployment device prior to radial expansion of the endoprosthesis.
Fig. 2b is a side view of the distal portion of the deployment device subsequent to radial expansion of the endoprosthesis adjacent the aneurysm.
Fig. 3a is a perspective view of an endoprosthesis having a plurality of polymer layers.
Fig. 3b is a cross-section through the endoprosthesis of Fig. 3a.
Fig. 4 is a cross-section through an endoprosthesis.
Fig. 5 is a perspective view of an endoprosthesis.
Fig. 6 is a cross-section of an endoprosthesis.
Fig. 7a is an endoprosthesis having a cover having a plurality of movable plates.
Fig. 7b illustrates a radially compressed configuration of several plates of the endoprosthesis of Fig. 7a.
Fig. 7c illustrates a radially expanded configuration of several plates of the endoprosthesis of Fig. 7a.
Fig. 8a is cover with a metallic film defining fenestrations configured to have minimal stress in a radially compressed state.
Fig. 8b illustrates the cover of Fig. 8a in a state of radial compression about midway between the radially compressed state of Fig. 8a and a fully expanded state.
Fig. 8c illustrates the cover of Fig. 8a in a state of radial expansion about that assumed in a body passage.
Fig. 9 is a cover with a metallic film defining fenestrations configured to have minimal stress in a radially expanded state within a body passage.

### DETAILED DESCRIPTION

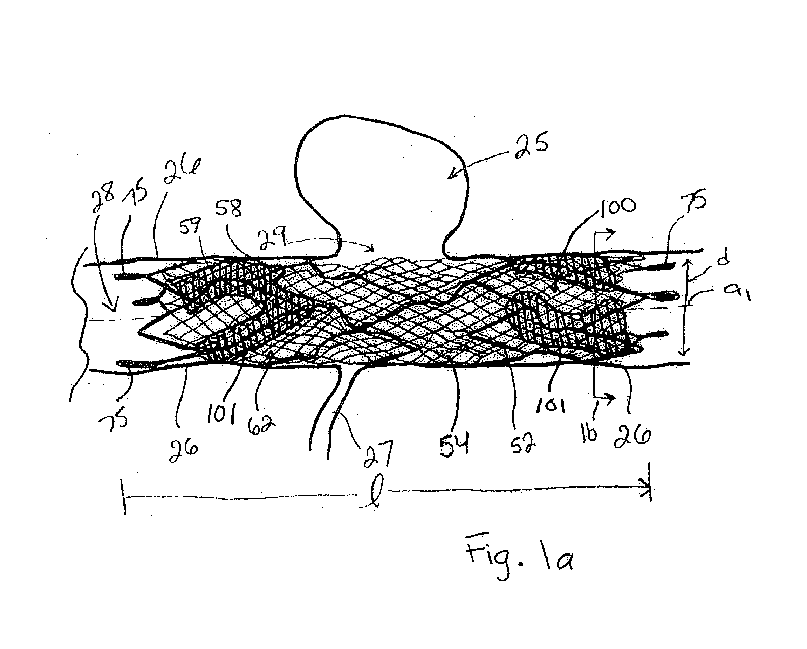

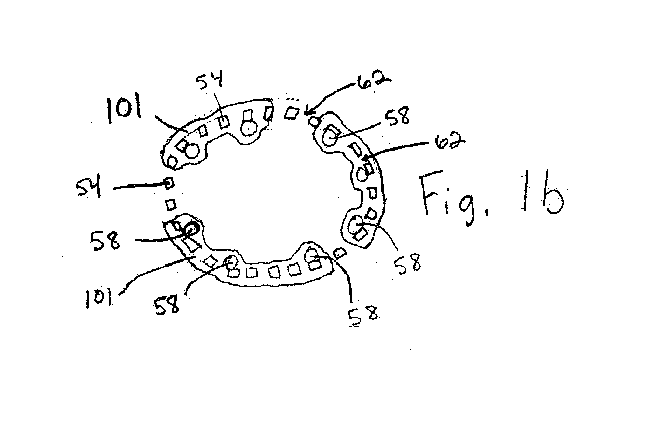

Referring to Figs. 1a and 1b, an endoprosthesis 100 is deployed within a body passage, e.g., within a vessel weakened by an aneurysm, e.g., an aneurysm 25 of a vessel 26 of a human brain. Endoprosthesis 100 includes a framework, e.g., a stent body 52, covered by a tubular member or cover 54, which are secured to one another by polymer layers 101. The stent body provides a relatively rigid framework that secures the endoprosthesis at the treatment site. The framework defines relatively large openings or fenestrations that contribute to the mechanical properties of the stent. The cover 54 is relatively thin and flexible and includes smaller fenestrations that contribute to the mechanical properties of the cover 54 and can occlude the fenestrations of the stent.

The endoprosthesis 100 modifies an amount or velocity of blood passing between vessel 26 and aneurysm 25. For example, prosthesis 100 can be deployed to divert, reduce or block blood flow between vessel 26 and aneurysm 25. The endoprosthesis can also reduce blood flow between vessel 26 and a feeder vessel 27. If so deployed, prosthesis 100 may sufficiently reduce blood flow to allow clotting or other healing processes to take place within aneurysm 25 and/or opening 29. Tubular member 54 can provide a greater attenuation of the blood flow into the aneurysm 25 than stent body 52 alone. Endoprosthesis 100, however, can allow some flow to pass between vessel 26 and aneurysm 25 even while providing flow diversion and/or reduction in flow. Prosthesis 100 can also (or alternatively) allow blood to pass between vessel 26 containing the prosthesis and adjacent vessels, e.g., feeder vessel 27, while still providing reduced flow with respect to the aneurysm.

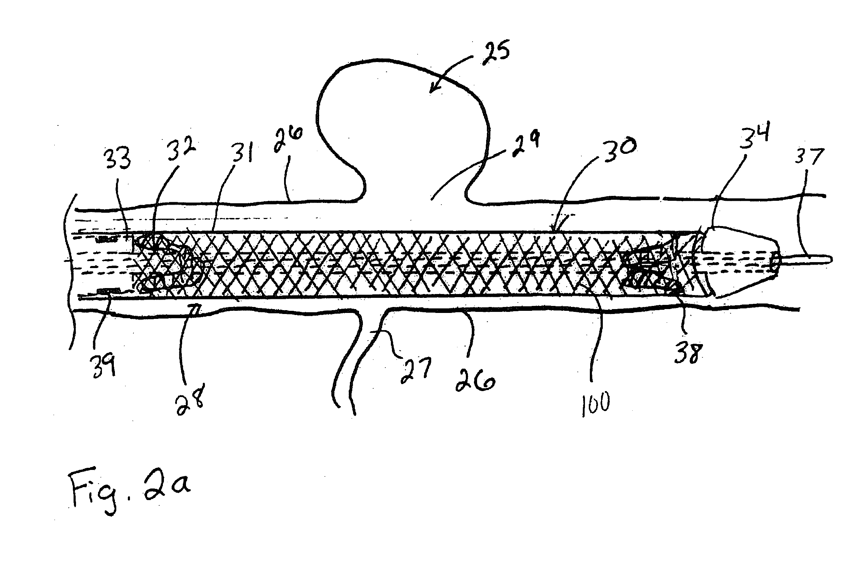

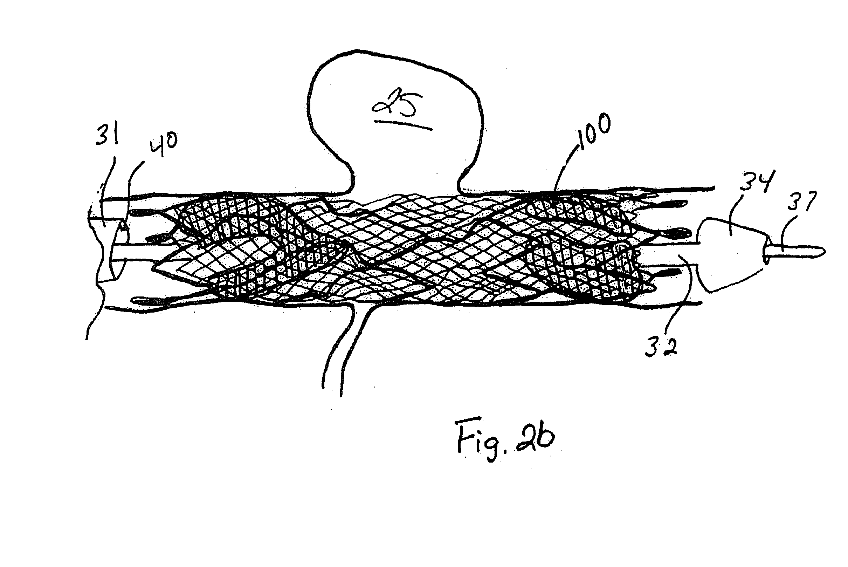

Referring to Figs. 2a and 2b, endoprosthesis 100 is deployed to aneurysm 25 using a deployment device 30, such as a catheter that can be threaded through a tortuous anatomy. The device 30 includes a retractable outer sheath 31 and an inner catheter 32. Device 30 is introduced over a guide wire 37 extending along the interior 28 of vessel 26. During introduction, the endoprosthesis 100 is radially compacted between outer sheath 31 and inner catheter 32 adjacent a distal opening 40 of the outer sheath.

Referring particularly to Fig. 2b, the outer sheath 31 is retracted upon reaching the desired deployment site, e.g., aneurysm 25. In some embodiments, endoprosthesis 100 self-expands by its own internal elastic restoring force when the radially restraining outer sheath is retracted. Alternatively, or in combination with self-expansion, deployment of prosthesis 100 may include use of a balloon or other device to radially expand prosthesis 100 within vessel 26. After deploying the endoprosthesis, the inner catheter 32 and guide wire 37 are withdrawn from vessel 26. Suitable delivery systems include the Neuroform, Neuroform2, and Wingspan Stent System available from Boston Scientific Target Therapeutics, Fremont, CA. In embodiments, the outer sheath and/or inner catheter includes a reinforcing member to respectively resist elongation or compression as the outer sheath is withdrawn. Such reinforcing members include polymer shafts, braids, and coil structures.

Upon expansion, the endoprosthesis assumes a shape and radial extent generally coextensive with an inner surface of the vessel 26, e.g., a tubular shape centered about a longitudinal axis a1 of the prosthesis (Fig. 1a). Depending upon the application, prosthesis 100 can have a diameter d of between, for example, 1 mm to 46 mm. In certain embodiments, a prosthesis for deployment within a vessel at an aneurysm can have an expanded diameter d of from about 2 mm to about 6 mm, e.g., about 2.5 mm to about 4.5 mm. Depending upon the application, prosthesis 100 can have a length along axis a1 of at least 5 mm, at least 10 mm, e.g., at least about 30 mm. An exemplary embodiment has an expanded diameter of about 3.5 mm and a length of about 15 mm. In embodiments, the stent body has a closed cell framework, an open cell framework, a helical framework, a braided framework, or combination thereof.

The cover can be fixed to the stent by, e.g. fasteners. Attachment techniques include brazing, welding or attachment with a filament, rivots or grommets, or crimping, or adhesive. In some embodiments, the tubular member differs from a fabric at least in that the tubular member lacks fibers that can be pushed apart to receive a filament as by sewing a fabric. Accordingly, the fenestrations can be formed prior to the process of passing the filament through the tubular member. Fenestrations that receive the filaments can be formed by, e.g., etching, laser cutting, or a photolithographic process. Attachment techniques are described in USSN _, titled MEDICAL DEVICES INCLUDING METALLIC FILMS AND METHODS FOR MAKING SAME, attorney Docket No. 10527-566001, filed contemporaneously herewith and incorporated herein by reference.

The cover is formed of a thin film that exhibits advantageous properties such as strength, toughness, and flexibility by selection of the composition of the film, processing techniques, and mechanical configuration. For example, in particular embodiments, the film is a vapor-deposited material composed of a nickel-titanium alloy having a strength additive, e.g. chromium. The film has a thickness of about 50 µm or less, e.g. about 4-35 µm, and includes fine fenestrations, which facilitate collapsing the film to small diameter for delivery into the body and expansion at the treatment site, while impeding blood access to the aneurysm. In particular embodiments, the film is processed to modify dislocations, which contribute to strength and toughness of the thin film.

Deposited materials, e.g., metallic films, are formed by depositing film constituents from a suspended state, e.g. in a vapor or a vacuum onto a surface. In embodiments, the constituents are suspended, e.g. by bombarding, heating or sputtering a bulk target. The suspended constituents deposit on a substrate to form the film. Deposited films can exhibit highly uniform thickness and microstructure in very thin films, e.g. about 50 µm or less, e.g. 4-35 µm. Deposition techniques include sputter deposition, pulsed laser deposition, ion beam deposition and plasma deposition. Suitable deposition processes are described in Busch et al. U.S. 5,061,914, Bose et al. U.S. 6,605,111, Johnston U.S. 6,533,905, and Gupta et al. U.S. 2004/0014253, the entire contents of all of which are hereby incorporated by reference.

In particular embodiments, the deposited film is an alloy that includes nickel and titanium, and a strength additive or additives, which modify a mechanical property, e.g., a hardness or elasticity, of the film. In particular embodiments, the film is a tertiary alloy that has substantially no other components besides nickel, titanium, and additive present in an amount greater than 1%, 0.5% or 0.1% or less than 20%, 10%, or 5% by weight of the film. The film may consist essentially of nickel, titanium, and chromium. In embodiments, the deposited film includes between 54 and 57 weight percent nickel with the balance composed essentially of titanium and chromium. In some embodiments, a ratio of a weight of chromium of the film to a combined weight of nickel, titanium, and chromium of the film is at least 0.001, at least 0.002 e.g., at least 0.0025. The ratio of the weight of chromium of the film to the combined weight of chromium, nickel, and titanium of the film can be 0.02 or less, 0.01 or less, e.g., 0.0075 or less. The ratio of the weight of chromium to the combined weight of chromium, nickel, and titanium of the film can be about 0.0025. In embodiments, the alloy exhibits superelastic or pseudo-elastic properties. Superelastic or pseudo-elastic metal alloy, as described, for example, in Schetsky, L. McDonald, "Shape Memory Alloys," Encyclopedia of Chemical Technology (3rd ed.), John Wiley & Sons, 1982, vol. 20. pp. 726-736; and commonly assigned U.S.S.N. 10/346,487, filed January 17, 2003.

A cover of deposited metal film contributes to desirable properties of an endoprosthesis. For example, as discussed above, cover 54 contributes to a flow diversion or reduction function. In some embodiments, a configuration and mechanical properties of the metallic film enhance the ability of the cover to withstand significant radial compression during deployment yet provide desirable properties in situ. An endoprosthesis can also include polymer layers, which, alone or in cooperation with a cover, contribute to properties of the endoprosthesis. Some polymer layers provide a mechanical function such as by securing a cover and stent body together or modifying surface properties of a metallic film, e.g., a lubricity or a roughness thereof. In embodiments, a polymer modifies a radial force exerted by the endoprosthesis against a body passage. Some polymers lend biological functionality to the endoprosthesis. For example, a polymer may improve biocompatibility, enhance cell growth, or provide a pharmacological function, e.g., release of a therapeutic agent. Embodiments of endoprostheses including covers having a metallic film are now described.

Returning to Figs. 1a and 1b, polymer layers 101 have a pattern that generally aligns with portions of the stent body, e.g., framework members 58,59 of the stent body. Fig. 1b shows that polymer layers 101 envelope members 58 and cover 54. A securing function is provided by mechanical properties of the polymer, which prevent the stent body and cover from tearing completely apart. Despite securing the stent body and tubular member, polymer layer 101 can allow some relative movement between the stent body and tubular member. In embodiments, relative movement occurs during radial compression and expansion and provides tolerance for some differential length changes, e.g., foreshortening, between the stent body and tubular member.

Polymers can be selected to provide desirable mechanical or chemical properties. For example, highly elongatable or elastic polymers rather than rigid polymers can be used to allow relative movement between a stent body and cover. In some embodiments, a layer of the polymer can have an elongation at break of at least 500%, at least 800%, at least 900%, or at least 1000%. A layer of the polymer can have a tensile modulus of at least 10,000 psi, at least 50,000 psi, or at least 75,000 psi. A layer of the polymer has a tensile strength of at least 2,500 psi, at least 5,000 psi, at least 7,500 psi, or at least 10,000 psi.

In some embodiments, the polymer includes or is formed of a butyric acid derivative polymer, e.g., poly-4-hydroxybutyrate, poly-4-hydroxybutyrate, or poly-(3-hydroxybutyrate-co-4-hydroxybutyrate). The butyric acid derivative polymer film may have a tensile strength of at least about 7,500 psi, a tensile modulus of about 10,000 psi, and an elongation at break of about 1,000%. Exemplary butyric acid derivative polymers are available from Tepha, Inc. of Cambridge, MA and include TephELAST₃₁ and TephaFLEX. Such butyric acid derivative polymers can provide better elongation and strength than polytetrafluorethylene while also providing an amount of lubricity.

The polymer can include a urethane alone or in combination with one or more additional polymers, e.g., as a copolymer. Exemplary urethanes include, e.g., polyurethane, dispersions and/or emulsions including aqueous dispersions and/or emulsions such as NeoRez R-985 (aliphatic polycarbonate diol), NeoRez R-986 (aliphatic polycarbonate diol) from Astra-Zeneca, W830/048 (polycarbonate backbone), W830/092 (modified polycarbonate background), W830/140 (polycarbonate backbone) and W830/256 (polycarbonate background), from Industrial Copolymer Ltd., Bayhydrol 121 (anionic dispersion of an aliphatic polycarbonate urethane polymer in water and n-methyl-2-pyrrolidone with a tensile strength of 6,700 psi and an elongation at break of 150%) and Bayhydrol 123 (anionic dispersion of an aliphatic polycarbonate urethane polymer in water and n-methyl-2-pyrrolidone with a tensile strength of 6,000 psi and an elongation at break of 320%) from Miles Inc. (Bayer AG).

In some embodiments, the polymer includes both urethane and silicone, e.g., a polyurethane/silicon copolymer. Such polymers can be highly compressible and exhibit elongations before break of 400% or more. Polyurethane/silicon copolymers tend to provide good adherence to the endoprosthesis. Exemplary silicone-polyurethane copolymers include the Elast-Eon series of polymers, e.g., Elast-Eon 2A, Elast-Eon 2D, Elast-Eon 3A, Elast-Eon 3LH and Elast-Eon HF polymers, available from Aortech of Victoria, Australia.

Other exemplary polymers include, e.g., biocompatible, non-porous or semi-porous polymer matrices made of a fluoropolymer, e.g., polytetrafluoroethylene (PTFE) or expanded PTFE, polyethylene, natural nylon, aqueous acrylic, silicone, polyester, polylactic acid, polyamino acid, polyorthoester, polyphosphate ester, polypropylene, polyester, or combinations thereof.

In some embodiments, polymer layer 101 releases a pharmaceutically active compound, e.g., a therapeutic agent or drug. Polymers providing such a release function are described in U.S. Patent No. 5,674,242, U.S.S.N. 09/895,415, filed July 2, 2001, and U.S.S.N. 10/232,265, filed August 30, 2002. The therapeutic agents, drugs, or pharmaceutically active compounds can include, for example, anti-thrombogenic agents, antioxidants, anti-inflammatory agents, anesthetic agents, anti-coagulants, and antibiotics. Exemplary polymers for releasing pharmaceutically active compounds include natural nylon, polysaccharides such as for example, methyl cellulose, hydroxymethyl cellulose, hydroxyethyl cellulose, hydroxypropyl cellulose, hydroxy-propylmethyl cellulose, hydroxpropylethyl cellulose, sodium carboxymethyl cellulose, hyaluronic acid, chondroitin sulfate, chitosan, dextran, xanthan, gellan, alginic acid, jota carrageenan; polypeptides such as for example, collagen, gelatin, elastin, albumin; and synthetic polymers such as for example, poly(vinyl alcohol), poly(lactic acid), polyglycolic acid, polycaprolactone, polyanhydride, ethylene vinyl acetate (EVA) their copolymers and mixtures thereof.

Polymer layers 101 can be formed by contacting a cover and stent body with a flowable or sprayable polymer, such as by dip coating or spray coating. Upon curing, the polymer provides functionality, e.g., securement, to the endoprosthesis. In some embodiments, significant portions, e.g., all of a length of an endoprosthesis are contacted with polymer. Subsequently, portions of the polymer are removed, e.g., by laser ablation after curing. Polymer can be removed quite selectively if desired. For example, a polymer that initially occludes fenestrations of a cover can later be removed from some or all of the fenestrations while leaving polymer surrounding the fenestrations. In other embodiments, portions of the cover or stent body are protected from contact with the polymer, e.g., by a mask or temporary coating.

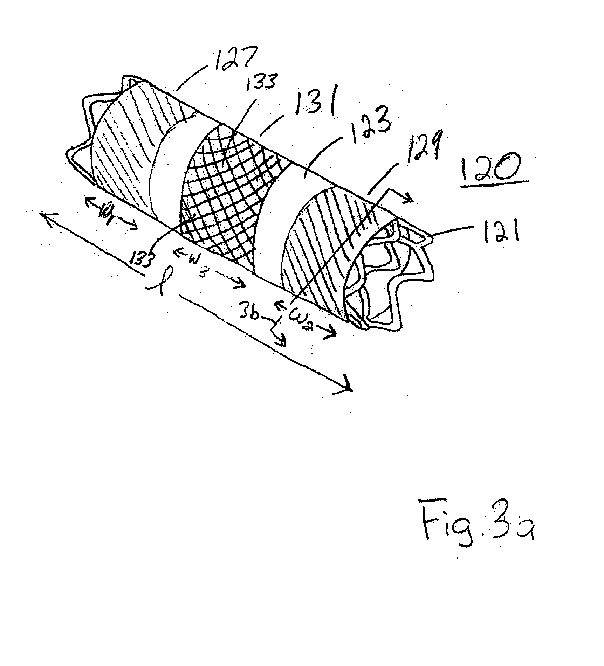

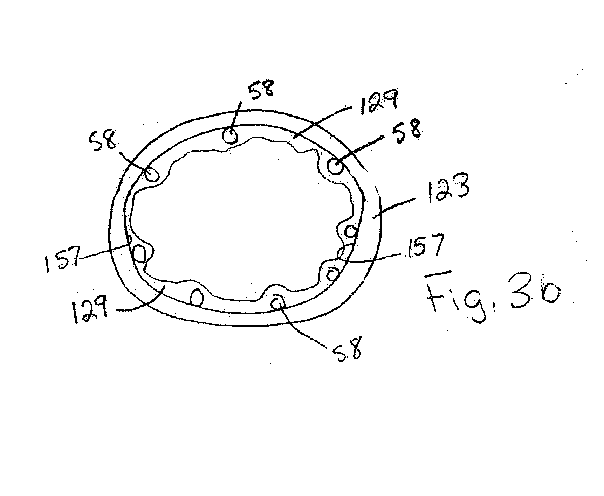

An endoprosthesis can include polymer layers configured differently from layers 101 to provide a securing function or other mechanical or biological functionalities. Referring to Figs. 3a and 3b, an endoprosthesis 120 includes a stent body 121 surrounded by a cover 123. Two polymer end portions 127,129 and a polymer central portion 131 extend generally circumferentially without following particular elements of the stent body.

In some embodiments, end portions 127,129 are located within the cover. As seen in Fig. 3b polymer layer 129 provides a securing function by adhering to an inner surface 157 of the cover. Polyurethane-silicone copolymers exhibit suitable adhesion properties yet allow some freedom of movement between the stent body and cover to tolerate differential length changes upon compression-expansion. Framework members 58 of a stent body are enveloped by the polymer layer, which, in the cross-section shown, is not present on an external surface of the prosthesis. Film 123 does not include fenestrations in the cross section shown and may include no fenestrations at all. In alternative embodiments, the stent body surrounds the cover with the polymer layer enveloping portions of the stent body and adhering to an external surface of the cover.

In some embodiments, end portions 127,129 have a sufficient thickness and material properties to increase (or decrease) a radial expansive force exerted by the end portions of the endoprosthesis. As seen in Fig. 1a, end portions of a deployed endoprosthesis engage vessel walls to either side of an aneurysm. Radial force exerted by the ends of the endoprosthesis prevents movement along the vessel without damaging the vessel walls. Polymer layers 127,129 can cooperate with a stent body and cover to provide an appropriate level of radial force, such as be resisting expansion of the stent body.

Polymer end portions 127,129 have respect widths w1,w2, which may be at least about 10% of the length of the endoprosthesis, e.g., at least about 20%, at least about 40%, e.g., at least about 60% of the length. The widths w1,w2 may be different. One of the polymer end portions is not be present in some embodiments. In some embodiments, polymer end portions are 25 µm thick or less, 20 µm thick or less, 15 µm thick or less, or 10 µm thick or less. The polymer can be formed of a plurality of individual layers, each having a thickness less than the total thickness of the polymer. For example, the polymer can be formed of a plurality of layers each having a thickness of 5 µm or less or 2 µm or less. The central polymer portion 131 has a width w3 configured to straddle an aneurysm or other treatment site. In some embodiments, central polymer portion 131 provides a permanent flow diversion or flow reduction function that cooperates with fenestrations 133 of the cover.

In some embodiments, polymer end portions 127,129 are located external to cover 123 and include a topography or chemical properties configured to enhance long-term engagement of the endoprosthesis and the vessel walls adjacent the treatment site. For example, the topography of the outer surface of the polymer layers can include a plurality of pores having a size sufficient to enhance cell in-growth. The polymer releases compounds to enhance such growth. The central polymer portion may also release drugs or other therapeutic agents.

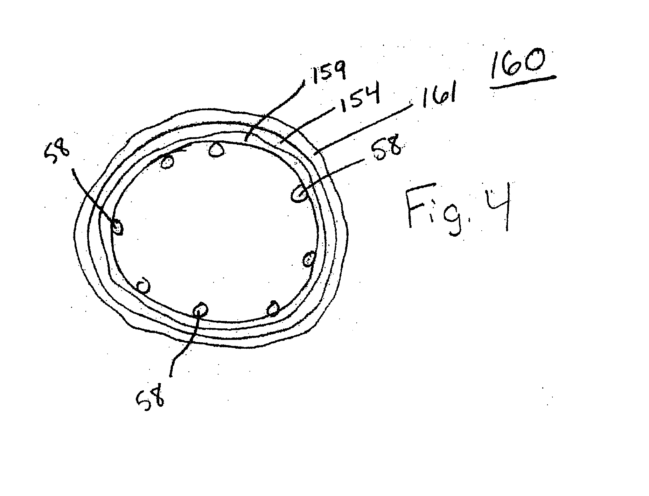

Referring to Fig. 4 an endoprosthesis 160 includes a composite cover comprising an interior polymer layer 159, a metallic film layer 154, and an exterior polymer layer 161. The composite cover surrounds a stent body with framework members 58. Layers 159,161 can be formed from a flowable composition of the polymer. In other embodiments, the metallic film is deposited, e.g., by vapor deposition, directly onto one of polymer layers 159 or 161. A polymer layer may itself be deposited from a vapor onto the metallic film. Alternative composites are also possible. For example, the layers may be reversed so that a polymer layer is sandwiched by two metallic layers.

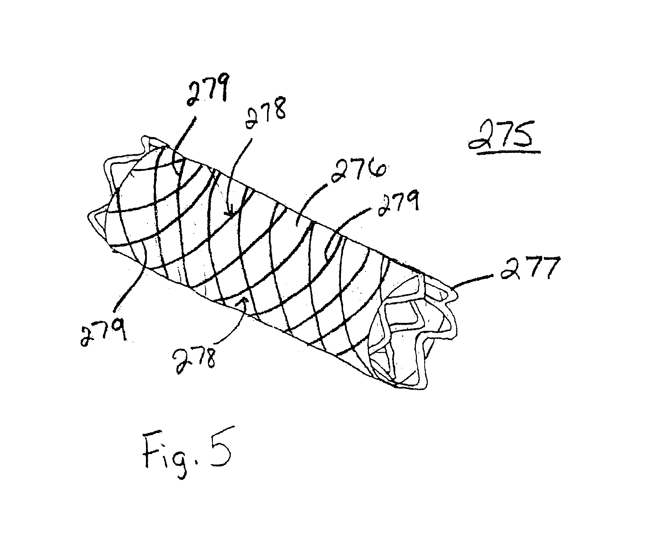

Referring to Fig. 5, an endoprosthesis 275 includes a patterned polymer layer 278, which modifies a radial force exerted by a stent body 277 and cover 154 of the endoprosthesis. Patterned polymer layer 278 is formed of a plurality of polymer strands 279 extending circumferentially with respect to the endoprosthesis 275. Each strand 279 defines a helix encircling an exterior of a cover 154. Strands defining opposed orientations cooperate to define a lattice structure of the patterned layer 278.

Strands 279 may be oriented fibers of a polymer having a high tensile modulus and tensile strength. In some embodiments, the strands are oriented fibers of a butyric acid derivative having a tensile modulus of at least 100,000 psi and a tensile strength of at least about 70,000 psi. Oriented fibers of TephaFLEX available from Tepha, Inc. are exemplary. The oriented fibers can guide and constrain radial expansion of the endoprosthesis. In such embodiments, the maximum expanded diameter of the deployed endoprosthesis may be less than a diameter attained in the absence of pattern 278.

Strands 279 may be formed of a polymer having a highly compressible polymer having a high elongation before break. Urethane-silicone copolymers such as from the Elast-Eon series of polymers from Aortech can provide such properties. For example, a polymer Elast-Eon 3LH from Aortech has a tensile modulus of about 1,000 psi and an elongation before break of about 650%. Such highly compressible and elongatable polymers can contribute positively to a radial force exerted by the endoprosthesis.

The polymer pattern 278 can be formed by spin coating strands 278 such as by extruding a polymer through a nozzle and rotating the endoprosthesis with respect to the nozzle. The extruded strands 279 typically have a thickness of about equal to or less than cover 154. In some embodiments, strands 279 may have a diameter of about 10 µm or less, e.g., about 2 µm or less.

The polymer bands can have a thickness less than that of the tubular member. For example, the polymer bands can be about 50% of a thickness of a thin film of the tubular member.

Although pattern 278 is shown disposed about an entire length of cover 154, a central portion, e.g., at least a central 30%, 40%, 60%, 80%, or 90% of the endoprosthesis 275 may lack a polymer pattern sufficient to substantially modify a radial expansive force of the endoprosthesis. For example, a central portion of the endoprosthesis can include a polymer that contributes to other properties, e.g., lubricity, fenestration occlusion, or therapeutic agent delivery without substantially altering a radial expansive force of the endoprosthesis.

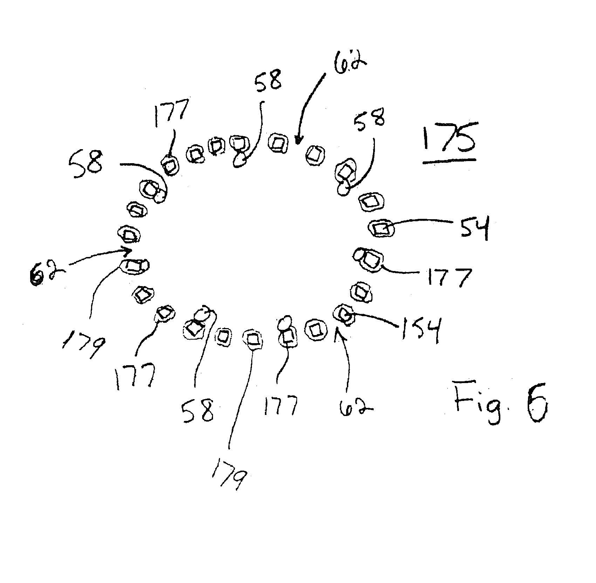

Referring to Fig. 6, an endoprosthesis 175 seen in cross-section includes a stent body having framework members 58 and cover 54 enveloped by a polymer layer 177, which provides a smoother outer surface than an untreated, deposited metallic film. Compared to the untreated film, the polymer layer 177 can have a smoother topography, an increased lubricity, a lower surface energy, improved mechanical properties, e.g., improved stretchiness or tear resistance, or combination thereof. For example, outer portions 179 of the cover 54 exhibit a lower coefficient of friction when translated with respect to the inner surface of a sheath used to deploy the endoprosthesis. Hence, during deployment, less force is required to begin withdrawing the sheath from the radially compressed endoprosthesis. In the embodiment shown, fenestrations 62 of cover 54 are not occluded by layer 177, which has a smaller thickness than the cover. For example, layer 177 may have a thickness of a few microns or less.

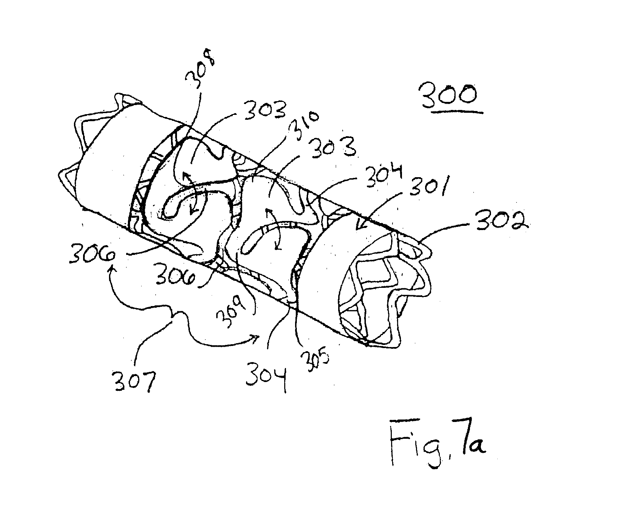

Referring to Fig. 7a, an endoprosthesis 300 includes a tubular member 301 having a plurality of plates 303, which spread apart upon radial expansion of the endoprosthesis. Because of the expansion, tubular member 301 can be radially compressed to a small diameter and then radially expand upon deployment to provide a substantially greater surface area than in the absence of spreading plates 303. Accordingly, endoprosthesis 300 can be delivered within a radially compact delivery device yet conform to the wall of a relatively larger diameter vessel upon deployment.

A central portion 307 of tubular member 301 includes a plurality of plates 303 connected by struts 304. A stent body 302 supports plates 303 and end portions of the tubular member. Adjacent plates 303 are separated by gaps 306 through which framework members 305 of stent body 302 can be seen. In other embodiments, the stent body does not extend between opposite ends of the endoprosthesis. Instead, two independent stent bodies provide a radial outward force to secure the prosthesis in a vessel.

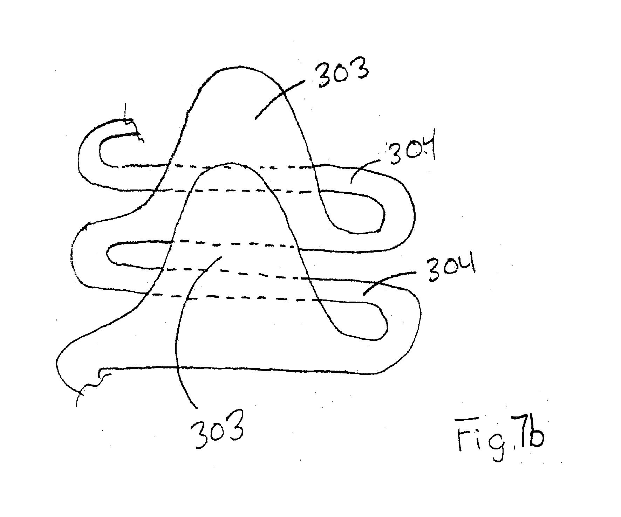

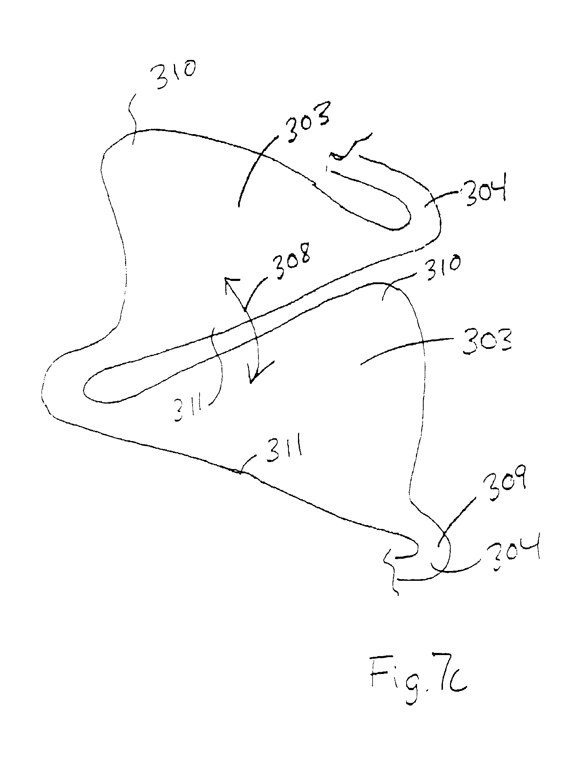

Referring also to Fig. 7b, adjacent plates 303 overlap when endoprosthesis 300 is radially compressed as for delivery along a blood vessel to an aneurysm site. Referring to Fig. 7c, plates 303 spread apart upon radial expansion increasing the effective surface area of central portion 307. Arrows 308 illustrate generally the relative movement of adjacent plates 303. Because plates 303 overlap when radially compressed and spread apart when radially expanded, central portion 307 tubular member 301 can define a greater surface area than would otherwise be possible without significantly changing the surface area of plates 303 themselves.

In some embodiments, at least 10%, at least 25%, at least 35%, at least 50%, or at least 70% of plates 303 are overlapped in the radially compressed state. Hence, the apparent surface area of endoprosthesis 300 can be significantly larger in the expanded state than in the radially compressed state. In some embodiments, 30% or less, 20%, or less, e.g., 10% or less of plates are overlapped in the radially expanded state. Some degree of overlap between plates can help limit a tendency of a plate to flex radially outwards or inwards in response to blood flow internal to or external to the deployed prosthesis. For example, a tip 310 of a plate can overlap or be overlapped by a base 311 of another plate (Fig. 7c).

A deposited metallic film can contribute desirable mechanical properties to plates and struts of the cover. For example, tubular member 300 can include a thin film, e.g., metallic film comprising nickel, titanium, and, optionally, a strength additive, e.g., chromium. An amount of strength additive may vary in different portions of the film. In some embodiments, elbows 309 include a different amount of strength additive than plates 303.

Plates and struts including a deposited metallic film can be formed with minimal thickness, e.g., about 50 microns or less, e.g., about 4 to about 35 microns. Struts 304 can include elbows 309 defining significant bends, e.g., 130° or more, 150° or more, or 180° or more. Elbows 309 can have a composition and/or cross-section different from plates 303. In some embodiments, elbows have a circular or oval cross-section whereas as plates 303 are substantially planar.

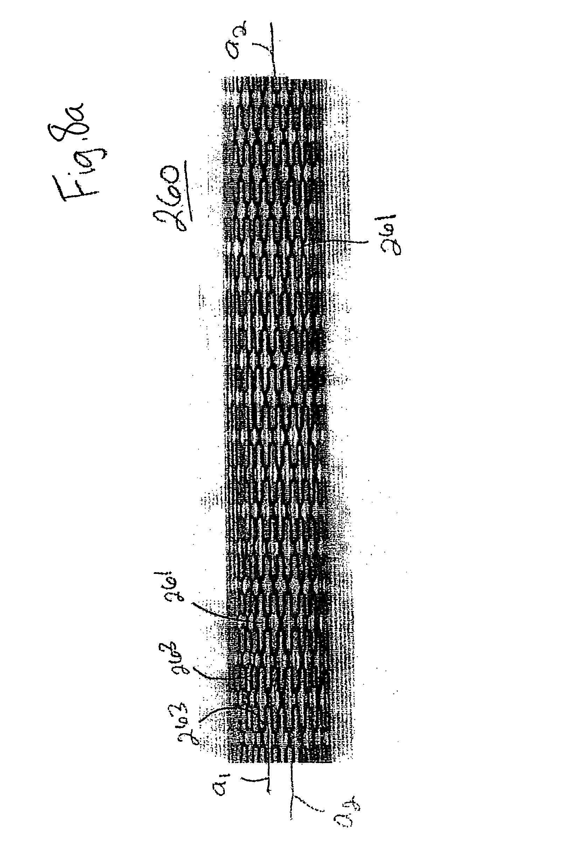

Referring to Fig. 8a, a metallic film 260 useful as a cover of an endoprosthesis includes a plurality of fenestrations 261 having minimal stress when radially compressed within a delivery device. Minimizing stress in the radially compressed state can reduce or prevent deformation, e.g., warping or kinking, of the film. Upon radial expansion, the fenestrations 261 may experience a relatively greater stress than an alternative fenestration configuration. However, because forces experienced by the radially expanded film tend to be more uniform, the film can tolerate radial expansion without deformation.

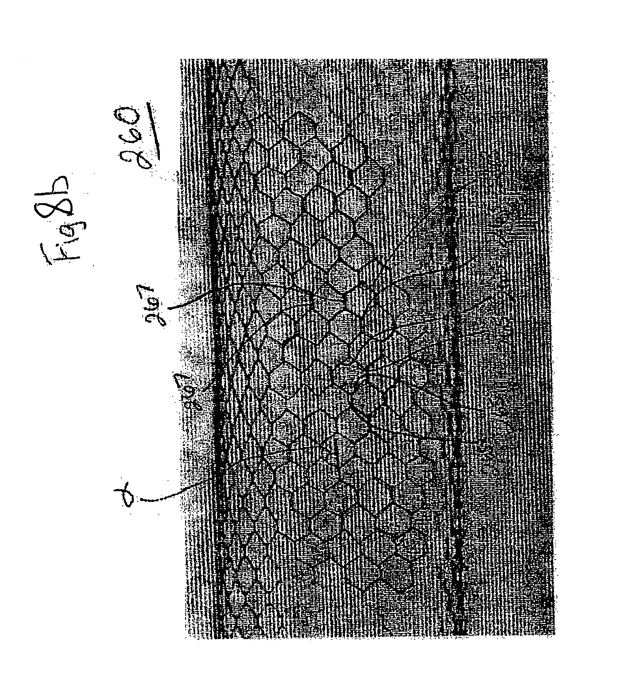

In a relatively unexpanded state (Fig. 8a), each fenestration 261 includes a plurality of parallel walls extending along a major fenestration axis al, which is parallel to a longitudinal axis a2 of an endoprosthesis that would receive the film 260 as a cover. Ends 263 of each fenestration are arcuate. Upon partial radial expansion (Fig. 8b), interior walls 265 adjacent the ends 263 spread apart defining a non-parallel angle α with the longitudinal axis a2. A pair of centrally located walls 267 remain parallel to one another. Accordingly, each fenestration 261 assumes a hexagon shape.

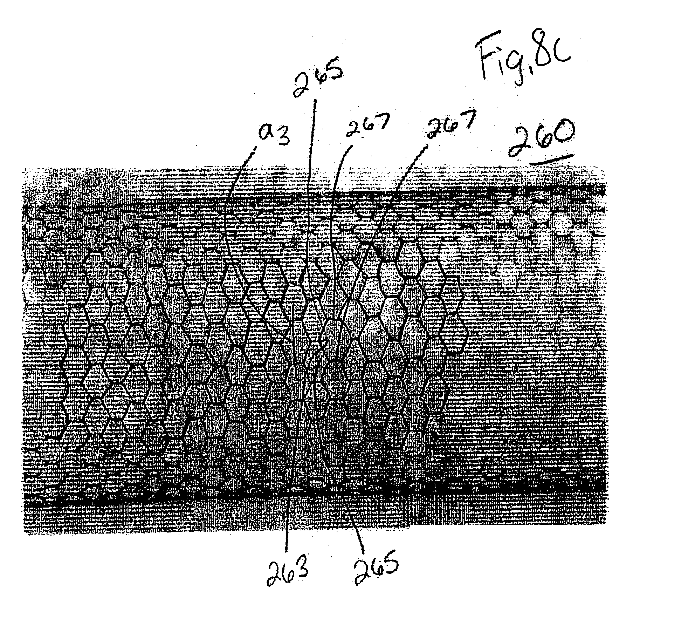

In a fully expanded state (Fig. 8c), e.g., at vessel size, walls 265 spread further apart and each fenestration 261 assumes an elongated hexagon having a major axis a3 aligned with a circumferential axis of the endoprosthesis. Walls 267 remain parallel to one another despite the circumferential elongation.

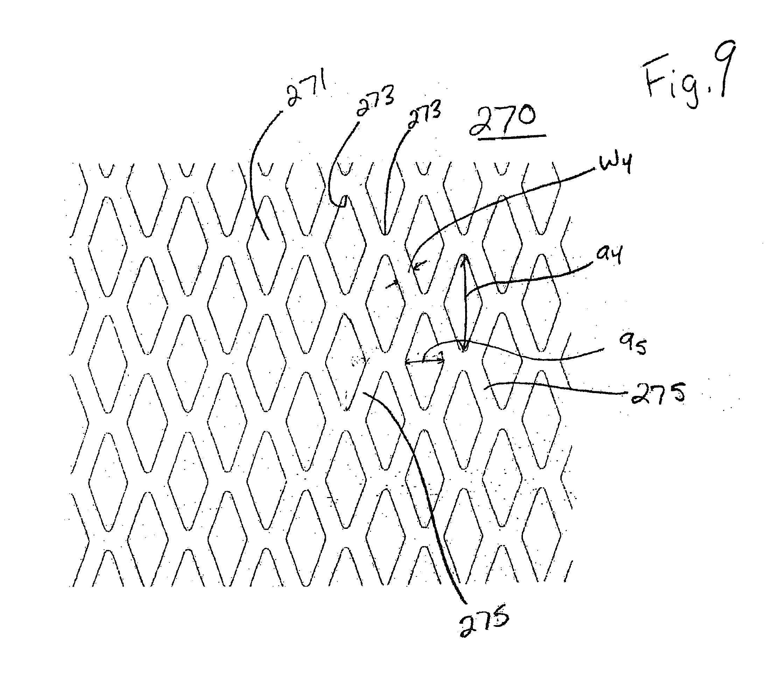

Referring to Fig. 9, a metallic film 270 useful as a cover of an endoprosthesis includes a plurality of struts 275, which define fenestrations 271 having minimal stress when radially expanded within a body passage, e.g., a vessel. In an unexpanded state, as shown, fenestrations 271 have a diamond shape defining a minor axis a5 and a major axis a4, which is aligned with a longitudinal axis of an endoprosthesis including the cover. A ratio (in the unexpanded state) of the major axis a4 to the minor axis a5 may be about 6 or less, about 5 or less, e.g., about 3 or less. A width w4 of metallic film struts 275 may be about 50 µm or less. A thickness of the film along a dimension normal to the film is less than the thickness of the struts and may be about 15 µm or less.

In addition to selecting a fenestration configuration that minimizes stress at a particular radial dimension, a cover can be shape set at a selected radial dimension. This shape set radial dimension may or may not match the radial dimension that minimizes stress of the fenestrations. A film can be shape set by, for example, setting the film at the selected radial dimension and heating the film to, e.g., about 500° C. In some embodiments, the film is shape set at a diameter about the same as or somewhat larger than an inner diameter of a delivery device sheath that surrounds the tubular member during implantation. In another embodiment, the film is shape set at a diameter about the same as or somewhat smaller than the inner diameter of a body passage to receive an expanded endoprosthesis. A stent body used with the cover may also be shape set to a selected radial dimension. A ratio of the shape set diameter of the cover 54 to the expanded diameter of stent body 52 in the absence of tubular member 54 may be about 1 or less, about 0.95 or less, or about 0.9 or less.

In other embodiments, a deposited metallic thin film and one or more polymer layers are useable as an endoprosthesis without a supporting stent. For example, an endoprosthesis without a supporting stent can include a deposited thin film formed of a selected alloy and one or more polymer layers to enhance radial and/or longitudinal strength. In embodiments, the deposited metallic film is in the shape of a tube of substantially uniform thickness. The metallic film can include a pattern of polymer layers or strands.

In the embodiment shown, endoprosthesis 100 has a generally tubular shape. In some embodiments, however, the endoprosthesis (or stent body 52 or tubular member 54 individually) has or includes other shapes such as conical, oblate, and branched. The endoprosthesis may have a closed end to form, e.g., a basket shape. Thin films, discussed above, composed of Ni-Ti-strength additive alloys and/or with modified microstructures, can be used in other applications. Examples include baskets, filters, catheters, guidewires, and medical balloons, such as an angioplasty balloon.

Other examples of endoprostheses including a thin film as well as related systems and methods are described in U.S. provisional patent application no. 60/549,287, filed March 2, 2004, which application is incorporated herein by reference.

An endoprosthesis may include a cover disposed externally to a framework as shown and/or internally of a framework. Endoprostheses having a cover including, e.g., a deposited thin film, disposed internally of a framework are described in U.S. patent application no. **/***,***, attorney docket no. 10527-567001, titled MEDICAL DEVICES INCLUDING METALLIC FILMS AND METHODS FOR MAKING SAME, and filed concurrently herewith, which application is incorporated herein by reference.

An endoprosthesis may include features to enhance a flexibility of the endoprosthesis as described in U.S. patent application no. **/***,***, attorney docket no. 10527-568001, titled MEDICAL DEVICES INCLUDING METALLIC FILMS AND METHODS FOR MAKING SAME, and filed concurrently herewith, which application is incorporated herein by reference.

The composition and/or fabrication method of a deposited thin film of an endoprosthesis may include features that enhance a strength or toughness of the film as described in U.S. patent application no. **/***,***, attorney docket no. 10527-570001, titled MEDICAL DEVICES INCLUDING METALLIC FILMS AND METHODS FOR MAKING SAME, and filed concurrently herewith, which application is incorporated herein by reference.

An endoprosthesis may include one or more filaments, e.g., wires, adapted to enhance mechanical properties of a deposited thin film as described in U.S. patent application no. **/***,***, attorney docket no. 10527-621001, titled MEDICAL DEVICES INCLUDING METALLIC FILMS AND METHODS FOR MAKING SAME, and filed concurrently herewith, which application is incorporated herein by reference.

Methods for loading an endoprosthesis into a delivery device and systems for delivering an endoprosthesis to a treatment site are described in U.S. patent application no. **/***,***, attorney docket no. 10527-569001, titled MEDICAL DEVICES INCLUDING METALLIC FILMS AND METHODS FOR LOADING AND DEPLOYING SAME, which application is incorporated herein by reference.

All publications, references, applications, and patents referred to herein are incorporated by reference in their entirety.

Other embodiments are within the claims.

### Embodiments

Although the present invention is defined in the attached claims, it should be understood that the present invention can also (alternatively) be defined in accordance with the following embodiments:
1. An endoprosthesis, comprising:
   a tubular framework having first end and second ends;
   a deposited metallic film generally coextensive with at least a portion of the framework, the metallic film having a thickness of less than about 50 µm; and
   a polymer layer securing the tubular framework and the deposited metallic film together.
2. The endoprosthesis of embodiment 1, wherein the deposited metallic film comprises deposited nickel and titanium.
3. The endoprosthesis of embodiment 1, comprising a plurality of polymer layers, each polymer layer having a configuration generally aligned with a portion of the tubular framework.
4. The endoprosthesis of embodiment 1, wherein the framework includes a plurality of framework members and the polymer layers envelope at least some of the framework members and at least a portion of the metallic film.
5. An endoprosthesis, comprising:
   a tubular framework having first end and second ends;
   a deposited metallic film generally coextensive with at least a portion of the framework, the metallic film having a thickness of less than about 50 µm; and
   at least one polymer strand extending circumferentially around the endoprosthesis.
6. The endoprosthesis of embodiment 5, wherein the deposited metallic film comprises deposited nickel and titanium.
7. The endoprosthesis of embodiment 5, comprising a plurality of polymer strands each extending circumferentially around the endoprosthesis.
8. The endoprosthesis of embodiment 7, wherein the plurality of polymer strands define a helical lattice.
9. The endoprosthesis of embodiment 5, wherein the endoprosthesis exerts a radial expansive force when deployed within a body passage and the at least one polymer strand contributes at least a portion of the radial expansive force.
10. The endoprosthesis of embodiment 5, wherein the polymer is a derivative of butyric acid.
11. The endoprosthesis of embodiment 5, wherein the polymer comprises a copolymer of urethane and silicone.
12. An endoprosthesis, comprising:
   a tubular member, at least a central portion of the tubular member comprising a pluralityofplates connected by struts, each of the plates being movable with respect to at least another plate, wherein, when the endoprosthesis is radially compressed for delivery along a body passage,
   at least some of the plates overlap another plate and, when the endoprosthesis is radially expanded within a body passage, an extent of the overlap decreases.
13. The endoprosthesis of embodiment 12, wherein the plates comprise a deposited metallic film.
14. The endoprosthesis of embodiment 13, wherein the deposited metallic film comprises deposited nickel and titanium.
15. An endoprosthesis configured to be deployed within a body passage by using a deployment device, the endoprosthesis being radially compressed within the deployment device and relatively radially expanded within the body passage, the endoprosthesis comprising:
   a deposited metallic film having a plurality of fenestrations, the fenestrations configured to have a lower stress in the radially compressed state than in the relatively radially expanded state.
16. The endoprosthesis of embodiment 15, wherein the endoprosthesis defines a longitudinal axis, each fenestration, in the radially compressed state, has a generally slit-like shape defined by a plurality of walls extending generally parallel to the longitudinal axis.
17. The endoprosthesis of embodiment 16, wherein, in the radially expanded state, at least some of the walls of each fenestration define an angle with respect to the longitudinal axis and at least some of the walls remain generally parallel with the longitudinal axis.

## Claims

1. An endoprosthesis, comprising:
a tubular framework having first end and second ends;
a deposited metallic film generally coextensive with at least a portion of the framework, the metallic film having a thickness of less than about 50 µm; and
at least one polymer strand extending circumferentially around the endoprosthesis.

2. The endoprosthesis of claim 1, wherein the deposited metallic film comprises deposited nickel and titanium.

3. The endoprosthesis of claim 1, comprising a plurality of polymer strands each extending circumferentially around the endoprosthesis.

4. The endoprosthesis of claim 3, wherein the plurality of polymer strands define a helical lattice.

5. The endoprosthesis of claim 1, wherein the endoprosthesis exerts a radial expansive force when deployed within a body passage and the at least one polymer strand contributes at least a portion of the radial expansive force.

6. The endoprosthesis of claim 1, wherein the polymer is a derivative of butyric acid.

7. The endoprosthesis of claim 1, wherein the polymer comprises a copolymer of urethane and silicone.

8. The endoprosthesis of claim 1, wherein a patterned polymer layer is formed of a plurality of polymer strands.

9. The endoprosthesis of claim 1 or 8, wherein the strands are oriented fibers of a butyric acid derivative having a tensile modulus of at least 100,000 psi and a tensile strength of at least about 70,000 psi.

10. The endoprosthesis of claim 1 or 8, wherein the oriented fibers guide and constrain radial expansion of the endoprosthesis.

11. The endoprosthesis of claim 1 or 8, wherein the polymer comprises a copolymer of urethane and silicone which has a tensile modulus of about 1,000 psi and an elongation before break of about 650%.

12. The endoprosthesis of claim 1 or 8, wherein the strands have a diameter of about 10 µm or less, or about 2 µm or less.

13. The endoprosthesis of claim 8, wherein the polymer pattern is formed by

14. The endoprosthesis of claim 8, wherein a central portion lacks a polymer pattern.

15. The endoprosthesis of claim 14, wherein at least a central 30%, 40%, 60%, 80%, or 90% of the endoprosthesis lack a polymer pattern.
